(19) Europäisches Patentamt
European Patent Office
Office européen des brevets

(11) Veröffentlichungsnummer: **0 336 305**
**A1**

(12) # EUROPÄISCHE PATENTANMELDUNG

(21) Anmeldenummer: **89105659.0**

(22) Anmeldetag: **31.03.89**

(51) Int. Cl.[4]: **C07C 103/84 , C07C 101/28 , C07C 102/00 , C07C 99/00 , C07D 263/42 , C07D 307/54 , C07D 333/24 , A61K 31/195 , A61K 31/42**

(30) Priorität: **02.04.88 DE 3811256**

(43) Veröffentlichungstag der Anmeldung: **11.10.89 Patentblatt 89/41**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Burger, Klaus, Prof. Dr.**
**Untere Dorfstrasse 33**
**D-8051 Kranzberg(DE)**
Erfinder: **Geith, Klaus, Dr.**
**Ziegeleistrasse**
**D-8074 Gaimersheim(DE)**
Erfinder: **Gaa, Karl**
**Zusam-Strasse 18**
**D-8901 Dinkelscherben(DE)**

(54) **Alpha-Trifluormethylaminosäure-Derivate, Verfahren zur Herstellung von alpha-Trifluormethylaminosäure-Derivaten und ihre Verwendung als Arzneimittel.**

(57) α-Trifluormethylaminosäure-Derivate werden hergestellt durch Umsetzung von 5-halogensubstituierten 4-Trifluormethyloxazolen mit β-γ-ungesättigten Alkoholen und Hydroxymethyl-aromaten bzw. -heteroaromaten.

EP 0 336 305 A1

## α-Trifluormethylaminosäure-Derivate, Verfahren zur Herstellung von α-Trifluormethylaminosäure-Derivaten und ihre Verwendung als Arzneimittel

Die vorliegende Erfindung betrifft α-Trifluormethylaminosäure-Derivate, Verfahren zur Herstellung von α-Trifluormethylaminosäure-Derivaten und ihre Verwendung als Arzneimittel.

Einige α-Trifluormethylalanin-aminosäure-Derivate sind bereits bekannt. In dem US-Patent 3,046,300 wird z.B. u.a. die Herstellung von Verbindungen der folgenden Formel beschrieben

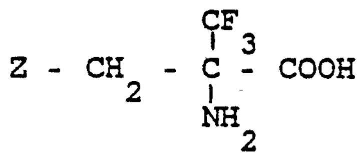

in der Z ein gegebenenfalls substituierter Phenylring ist. Der angegebene Syntheseweg umfaßt eine Vielzahl von Reaktionsschritten und ist offenbar für Synthesen, in denen der Substituent Z kein Phenylring ist, nicht geeignet.

Auch die Synthesevorschläge für einfache α-Trifluormethylaminosäuren über die in Christensen et al., Biochem. Biophys. Acta 74 (1963) 386 ff. und in Welch, Tetrahedron 43 (1987) 3123 berichtet wird, sind teilweise umständlich und nicht universell anwendbar.

Das es sich herausgestellt hat, daß α-Trifluormethylaminosäure-Derivate hochspezifische, in der Regel irreversible Enzyminhibitoren sein können

(a) A. Relimpio, J.C. Slebe, M. Martinez-Carrion, Biochem. Biophys. Res. Commun. 1975, 63, 625;
b) J.W. Keller, M.H. O'Leary, Biochem. Biophys. Res. Commun. 1979, 90, 1104;
c) R.H. Gordonsmith, M.J. Raxworthy, P.A. Gulliver, Biochem. Pharmacol. 1982, 31, 433;
d) J.T. Welch, Tetrahedron 43 (1987) 3123),

verstärkte sich das Bedürfnis nach einem einfachen, allgemein anwendbar Verfahren zur Herstellung der genannten Verbindungen. Mit der vorliegenden Erfindung wurde ein derartiges Verfahren gefunden.

Erfindungsgegenstand ist demzufolge ein Verfahren zur Herstellung von α-Trifluoralanin-Derivaten der Formel I und deren an der Carbonsäurefunktion und/oder an der Aminofunktion geschützten Formen

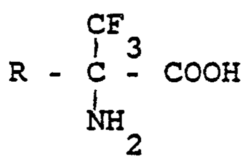

(I)

in der R ein gesättigter oder einfach oder mehrfach ungesättigter Kohlenwasserstoffrest mit mindestens 3 C-Atomen ist, der auch mit Heteroatomen und/oder verschiedenen funktionellen Gruppen substituiert sein kann, dadurch gekennzeichnet, daß man ein in 5-Stellung substituiertes 4-Trifluormethyloxazol der Formel II

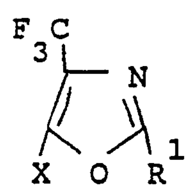

(II)

in der Z ein Halogen ist und $R^1$ ausgewählt ist aus der Gruppe Aryl, Heteroaryl, Alkyl und Cycloalkyl, bzw. deren mit schwach nucleophilen Heteroatomen und/oder funktionellen Gruppen substituierten Derivaten, mit einem β-γ-ungesättigten Alkohol umsetzt und mit der erhaltenen Verbindung eine Umlagerungsreaktion durchführt.

Für $R^1$ beispielhafte Reste sind insbesondere Phenyl, Halogenphenyl, insbesondere Fluorphenyl, Furyl, Thienyl, Methyl, Ethyl, Propyl, Butyl, Adamantyl, Cyclohexyl, Cycloheptyl, Cyclopentyl, Benzyloxy und Phenyloxy.

Das erfindungsgemäße Verfahren hat gegenüber den bislang bekannten Verfahren für die Synthese von

3,3,3-Trifluormethylaminosäure-Derivaten den Vorteil, daß

1. weniger Syntheseschritte notwendig sind;
2. es allgemeiner Natur ist und zur Herstellung α-$CF_3$-substituierter α-Aminosäuren angewendet werden kann, die sich von natürlichen oder von artifiziellen Aminosäuren ableiten;
3. es zur Herstellung aliphatischer, heterocyclischer, aromatischer und heteroaromatischer α-$CF_3$-substituierter α-Aminosäuren verwendet werden kann;
4. es für die Synthese di-, tri- und polyfunktioneller α-$CF_3$-substituierter α-Aminosäuren eingesetzt werden kann.

Erfindungsgegenstand sind ferner α-Trifluormethylaminosäure-Derivate der Formel I und deren an der Carbonsäurefunktion und/oder an der Aminofunktion geschützten Formen

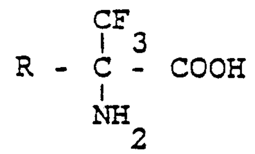

(I)

in der R ein gesättiger oder einfach oder mehrfach ungesättigter Kohlenwasserstoffrest mit mindestens 3 C-Atomen ist, der auch mit Heteroatomen und/oder verschiedenen funktionellen Gruppen substituiert sein kann, wobei die Verbindungen ausgenommen sind, die einen Rest R tragen, der eine -CHY-Z Gruppe ist und Z eine Phenyl- oder substituierte Phenylgruppe ist und Y nieders Alkyl, Wasserstoff oder eine OH Gruppe ist, sowie die Verbindungen der Formel I, in der R eine $-CH_2-CH_2-CH_2-NH_2$ Gruppe ist.

Unter Heteroatomen sind in dem vorstehenden Zusammenhang insbesondere Halogene, N, O, S und P zu verstehen. Geeignete funktionnelle Gruppen sind z. B. die -OR, -SR, -NRH, $-NR_2$, -COOR, mit R gleich H oder $C_1$-$C_5$-Alkyl, $-P(OH)_3$ oder $-SO_3H$ Gruppen.

Die Ausgangsverbindungen der Formel II für das erfindungsgemäße Verfahren sind durch Umsetzung von Heterodienen des Typs $(CF_3)_2$ C=N-CR=O oder $(F_2ClC)_2$ C=NCR=O mit $SnCl_2$ in guten Ausbeuten zugänglich (vgl. Ottlinger, Burger und Goth, Tetrahedron Lett. 1978, 5003 und Burger, Ottlinger, Goth und Firl, Chem. Ber. 115 (1982) 2494 und Zitate darin).

Die β-γ ungesättigten Alkohole sind, soweit nicht als Handelsprodukte verfügbar, auf literaturbekannte Weise darzustellen (vgl. Houben-Weyl, Bd, VI, Georg Thieme Verlag, Stuttgart, 1979). Bevorzugt wird das erfindungsgemäße Verfahren mit Allyl-, Propargyl- oder Benzylalkoholen durchgeführt.

Weiterhin bevorzugt wird das Verfahren zur Herstellung von Verbindungen der Formel III benutzt,

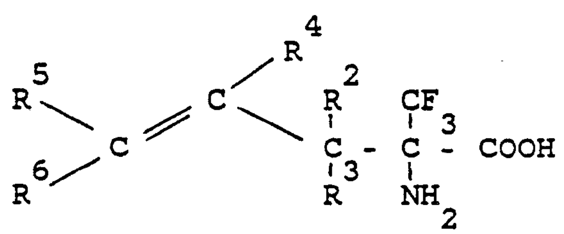

(III)

in der $R^2$ - $R^6$ Wasserstoff, geradkettige oder verzweigte Alkyl-, Alkenyl-, Alkinyl-, Acyl-, Aryl-, Aralkyl-, Heteroaryl- oder heterocyclische Gruppen mit bis zu 10 C-Atomen sein können, wobei die Substituenten Halogenatome tragen können und/oder durch funktionelle Gruppen wie OH-, -SH und/oder $-NH_2$ in geschützter oder ungeschützter Form substituiert sein können, und wobei die einzelnen Reste $R^4$ und $R^5$, $R^2$ und $R^3$, $R^5$ und $R^6$, $R^2$ und $R^5$, $R^2$ und $R^6$, $R^3$ und $R^5$ sowie $R^3$ und $R^6$ durch Verbrückung ein Ringsystem bilden können, das carbocyclischer, heterocyclischer und/oder aromatischer Natur sein kann, wobei jede einzelne Verbrückung bis zu 10 C-Atome aufweisen kann und zwei oder mehrere Ringsysteme anelliert oder miteinander verknüpft sein können und wobei die Verbrückungen substituiert sein können mit Alkyl- oder Alkenylgruppen mit bis zu 5 C-Atomen.

Allgemein läuft das erfindungsgemäße Verfahren ähnlich ab, wie für ein Beispiel für die Darstellung einer Verbindung des Typs der Formel III nachfolgend beschrieben.

Formelschema 1

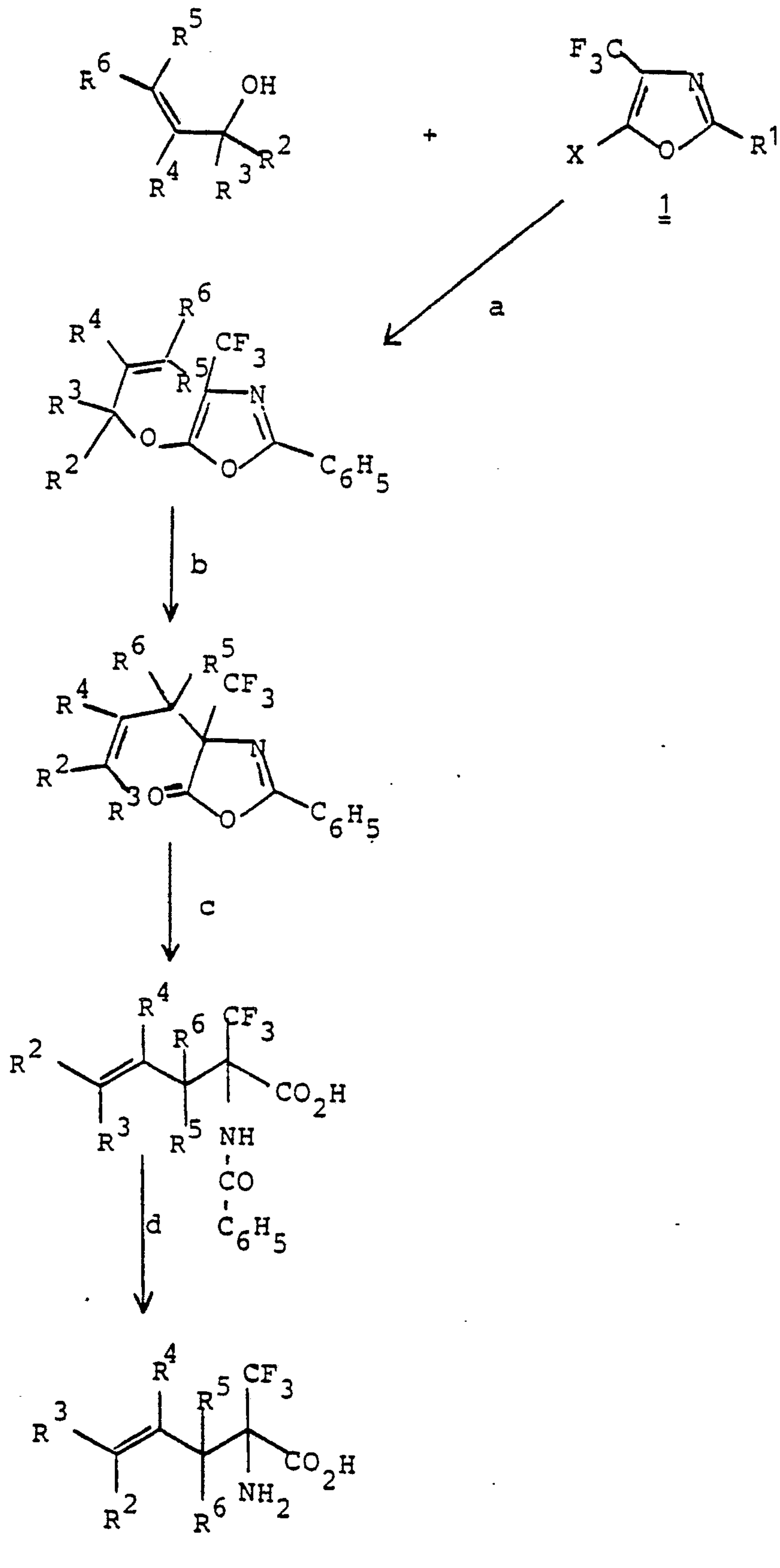

Aus mechanistischer Sicht beinhaltet die vorliegende Reaktionsfolge mehrere Teilschritte:

a) eine nucleophile Substitution des Halogenatoms in der Ringposition 5 des 4-Trifluormethyloxazols;

b) eine Hetero-Cope-Umlagerung;

c) eine Öffnung des Lactonrings;

d) eine Abspaltung der N-terminalen Schutzgruppe.

Zur Durchführung der Reaktion kann man des Oxazol in einem organischen Lösungsmittel, vorzugsweise in wasserfreiem Dioxan, Tetrahydrofuran oder Acetonitril lösen und den entsprechenden Alkchol zugeben, vorzugsweise in äquimolarer Menge. Der Reaktionsmischung sollte weiterhin eine Base zugefügt werden, vorzugsweise ein Alkalihydroxid oder die Hünig-Base (Diisopropylethylamin), besonders bevorzugt sind Natriumhydroxid, Kaliumhydroxid und die Hünig-Base. Die Reaktion erfolgt anschließend in der Regel spontan und exotherm. Der Reaktionsverlauf läßt sich zweckmäßigerweise mit Hilfe von $^{19}F$-NMR-Spektroskopie überwachen. Nach vollständigem Umsatz wird die Reaktionsmischung hydrolysiert, z.B. durch Zusatz von Wasser und nach vollständiger Öffnung des Lactonrings, was ebenfalls u.a. mit Hilfe von $^{19}F$-NMR-Spektroskopie erkennbar ist, zweckmäßigerweise eingeengt, gegebenenfalls bis zur Trockne. Der erhaltene Rückstand kann auf unterschiedliche Weise aufgearbeitet werden. Das optimale Aufarbeitungsverfahren kann von dem jeweiligen Reaktionsprodukt abhängig sein und ist gegebenenfalls empirisch zu ermitteln. In vielen Fällen ist es zweckmäßig, den Rückstand mit einer Wasser/Ether-Mischung, besonders bevorzugt im Verhältnis ca. 1:1, aufzunehmen und die Etherphase mehrmals mit Wasser zu extrahieren. Die vereinigten Wasserphasen können durch Zusatz von Säure vorzugsweise Mineralsäure sauer gestellt werden, z.B. durch Zugabe von HCl bis auf einen pH-Wert von ca. 1 - 2.

Anschließend wird zweckmäßigerweise mit einem organischen Lösungsmittel, vorzugsweise Ether mehrmals extrahiert. Nach Trocknung der Extrakte mit geeigneten Trockenmitteln, z.B. mit $NA_2SO_4$ oder mit $CaCl_2$, kann des erhaltene Produkt gegebenenfalls weiter gereinigt werden, z.B. durch chromatographische Methoden oder, vorzugsweise nach Entfernen des Lösungsmittels, durch Umkristallisation. Die Abspaltung der N-terminalen Schutzgruppe kann bei dieser Reaktion, wie auch bei den weiteren nachfolgend beschriebenen, z.B. durch Kochen mit konzentrierter HCl oder HBr/Eisessig erfolgen (vgl. z.B. Houben-Weyl, Bd. XV/1 u. 2, insbesondere XV/1 S. 198 ff, Georg Thieme Verlag, Stuttgart (1974)).

Das erhaltene Produkt kann anschließend unterschliedlichen Derivatisierungsreaktionen unterzogen werden. Als Angriffspunkt für Derivatisierungsreaktionen bietet sich insbesondere die C=C-Doppelbindung an, die Derivatisierungsreaktionen, wie z.B. im Organikum, VEB Deutscher Verlag der Wissenschaften, 15. Auflage, Berlin 1977, S. 308 ff. beschrieben, zugänglich ist, gegebenenfalls unter Anwendung der Schutzgruppentechnik für die Aminosäurefunktionen, wie z.B. von A. Hubbuch und E.F. Büllerbach in Merck Kontakte (Informationsschrift der Firma Merck, Darmstadt) 31/79 und 11/80 beschrieben. Die oxidative Spaltung der C=C-Doppelbindung führt zu $\alpha$-Trifluorsubstituierten Asparaginsäure-Derivaten.

Ganz besonders geeignet ist das Verfahren zur Herstellung von Verbindungen der Formel III, in der $R^2$ bis $R^6$ Wasserstoff, geradkettige oder verzweigte Alkyl- oder Alkenylgruppen mit 1 - 5 C-Atomen oder eine Arylgruppe sein können, insbesondere zur Herstellung von Verbindungen der Formel III, in denen die Substituenten $R^2$ bis $R^6$ Wasserstoff oder geradkettige oder verzweigte Alkyl- oder Alkenylgruppen mit 1 - 3 C-Atomen sind.

Weiterhin eignet sich das Verfahren besonders zur Herstellung solcher Verbindungen der Formel III, in denen mindestens eine Verbrückung zwischen den Substituenten $R^2$ bis $R^6$ besteht, wobei die Verbrückung bis zu 6 C-Atome, eine oder mehrere Doppelbindungen aufweisen kann, eines oder mehrere der Brücken-C-Atome durch N, O oder S und die Verbrückung durch einen oder mehrere Alkyl-, Alkenyl oder Arylsubstituenten mit bis zu 5 C-Atomen substituiert sein können. Die betreffenden zum Einsatz kommenden $\beta$-$\gamma$-ungesättigten Alkohole hätten z.B. die folgenden schematischen Formeln, in denen die Ringsysteme die genannten Bedeutungen haben können:

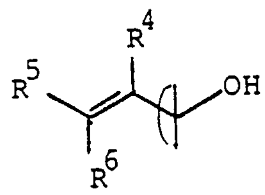

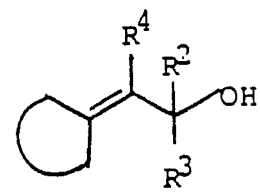

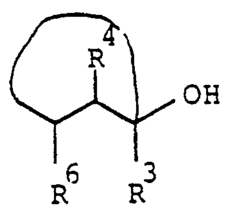

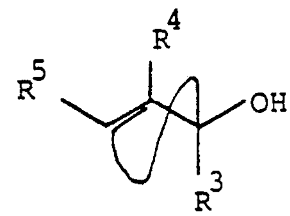

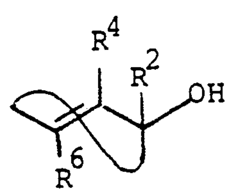

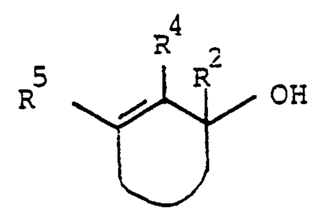

Insbesondere eignet sich das Verfahren zur Herstellung von Verbindungen, in denen die Verbrückung der Formel IV entspricht

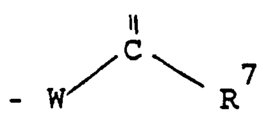

(IV),

in der $R^7$ Wasserstoff oder eine Alkylgruppe mit bis zu 4 C-Atomen und W $CH_2$, NH, O oder S darstellt.

Die Durchführung der betreffenden Reaktion erfolgt in gleicher Weise wie bereits beschrieben, wobei ein Allylalkohol eingesetzt wird, dessen Doppelbindung in das betreffende cyclische, aromatische oder heteroaromatische System integriert ist.

Im Falle der Umsetzung von Oxazolen der Formel II mit heterocyclischen Alkoholen des Typs

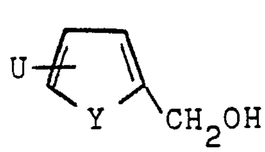

wobei Y = NR (R = H oder $C_1$-$C_6$-Alkyl oder Aryl), O oder S ist und U ein an den Heterocyclus gebundener Substituent ist mit der gleichen Bedeutung wie $R^2$-$R^6$, erfolgt nach der Cope-Umlagerung ein zusätzlicher Reaktionsschritt, nämlich eine prototrope Wanderung unter Rearomatisierung, so daß die entsprechenden Methylderivate entstehen.

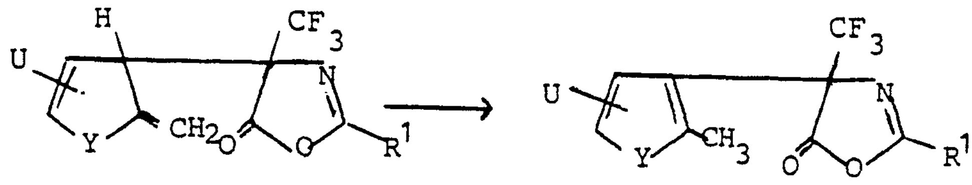

Eine weiter Verbindungsgruppe zu deren Herstellung das erfindungsgemäße Verfahren besonders geeignet ist, hat folgende Formel

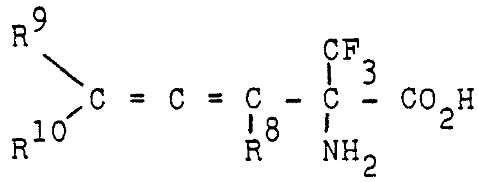

(V)

.

Die Substituenten $R^8$ bis $R^{10}$ sind Wasserstoff, geradkettige oder verzweigte Alkylreste mit bis zu 10 C-Atomen, heterocyclische Reste oder Aryl- oder Heteroarylreste, die mit Halogenatomen und/oder mit den mit Schutzgruppen versehenen Funktionen -OH, -SH, $-NH_2$, $-CO_2H$, $P(OH)_3$ und/oder $-SO_3H$, substituiert sein können, wobei die Reste $R^9$ und $R^{10}$ auch zusammen eine Verbrückung bilden können.

Die Darstellungsreaktion verläuft nach folgendem Formelschema:

Formelschema 2

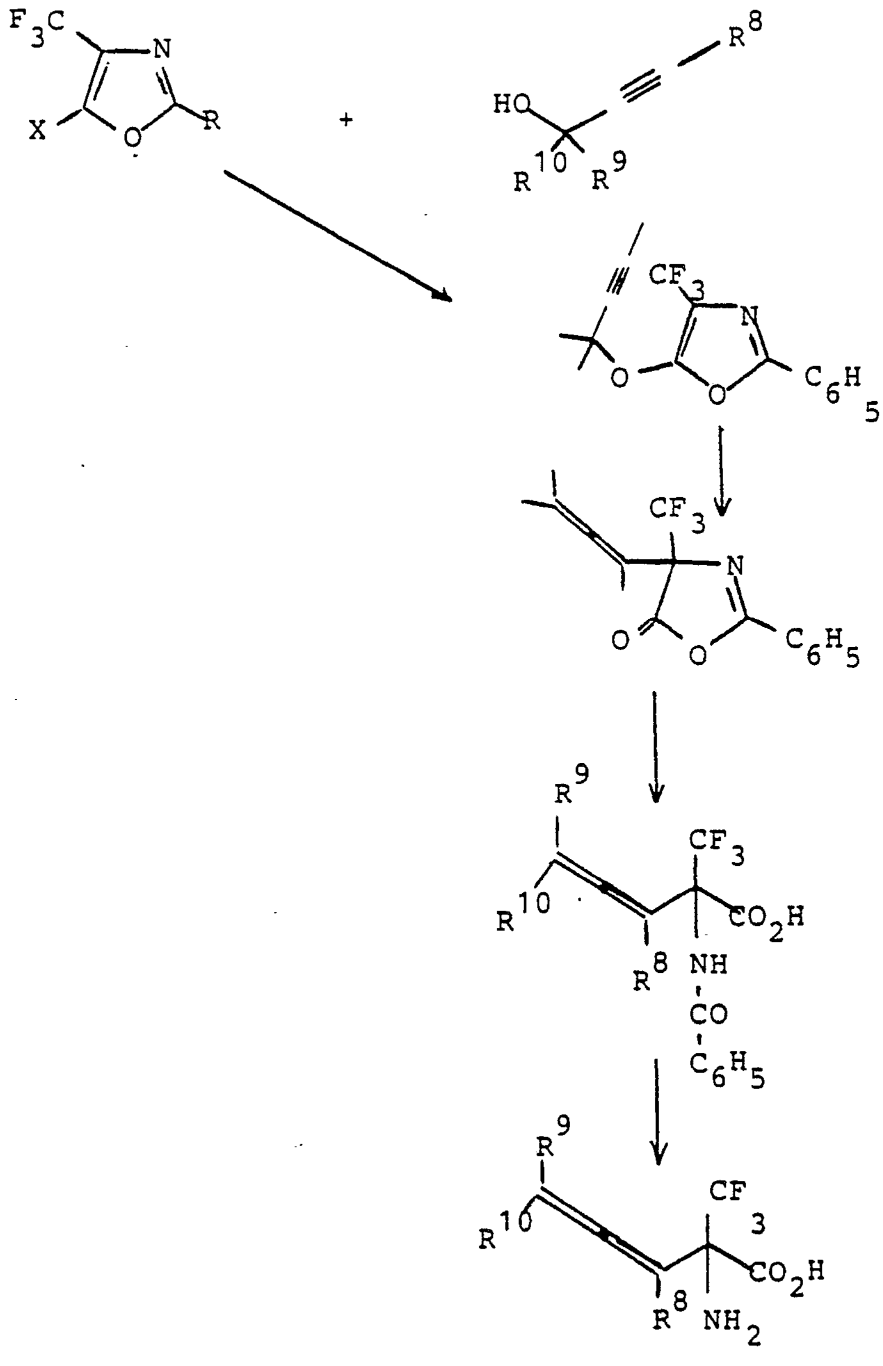

Die Reaktions- und Aufarbeitungsbedingungen dieser Umsetzung des Oxazols mit einem Propargylalkohol sind die gleichen, wie bei der zuvor beschriebenen Umsetzung mit einem Allylalkohol.

Weiterhin ist das erfindungsgemäße Verfahren besonders geeignet zur Herstellung von Verbindungen der Formel VI

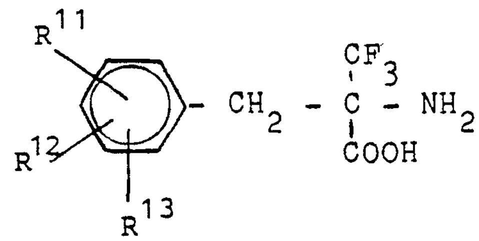

(VI)

in der $R^{11}$, $R^{12}$ und $R^{13}$ unabhängig voneinander Wasserstoff, Fluor, Chlor, Brom, Jod, Nitro-, Alkoxy-, Alkylgruppen mit bis zu 5 C-Atomen oder gegebenenfalls substituierte Phenylgruppen sein können, wobei zwei dieser Substituenten auch zusammen ein Ringsystem bilden können.

Die Umsetzung des Oxazols mit den entsprechenden Benzylalkoholen verläuft nach den nachstehenden beispielhaften Reaktionsschemata:

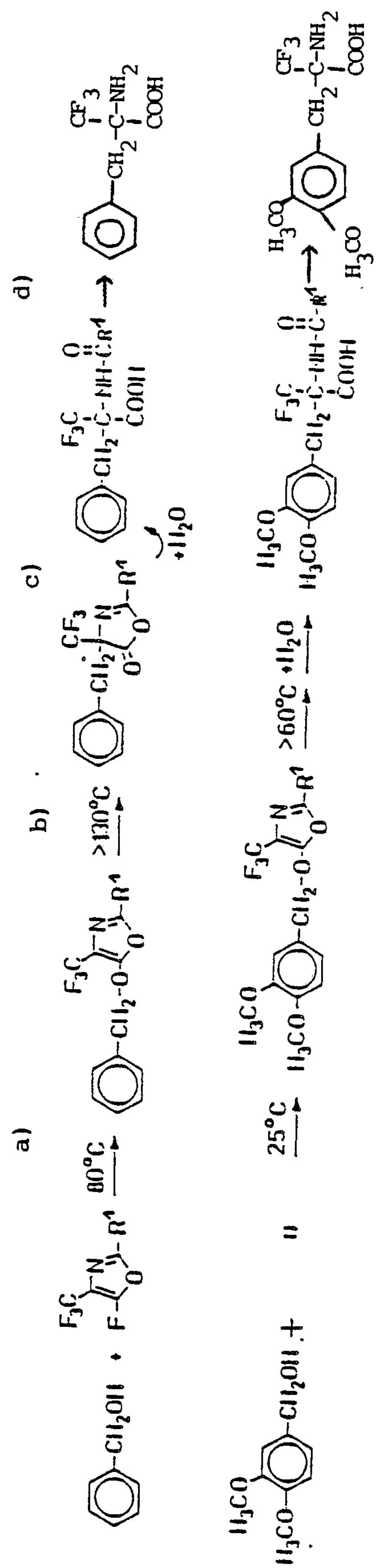
a)
80°C
b)
>130°C
c)
+H2O
d)
25°C
>60°C +H2O

Aus mechanistischer Sicht handelt es sich bei der vorbeschriebenen Reaktion um folgende Reaktionstypen:

a) einer nucleophilen Substitution des Halogenatoms in der Ringposition 5 des 4-Trifluormethyl-oxazols;

b) einer 1,3-Benzylgruppen-Wanderung;

c) einer Öffnung des Lactonringes;

d) einer Abspaltung der N-terminalen Schutzgruppe.

Der erste Reaktionsschritt erfolgt in gleicher Weise wie für die Umsetzung der Oxazole mit Allylalkoholen beschrieben, wobei es sinnvoll sein kann, die Reaktionsmischung zu erwärmen. Ob und auf welche Temperatur zu erwärmen ist, hängt von dem Substitutionsmuster des Benzylalkohols ab; die Erhitzungstemperatur, die in der Regel unter 100° C liegt, ist empirisch zu ermitteln. Substituenten, die die Elektronendichte am Benzolkern erhöhen, setzen die notwendige Temperatur herab.

Das Produkt, das nach dem 1. Reaktionsschritt anfällt, ist eine isolierbare neue Verbindung. Erfindungsgegenstand sind demzufolge auch Verbindungen der Formel VII

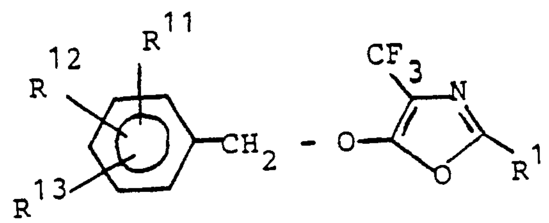

(VII)

in der $R^1$ die zu Formel (II) genannten Bedeutungen hat und die Substituenten $R^{11}$ bis $R^{13}$ die im Zusammenhang mit der Formel VI genannten Bedeutungen haben.

Die Reinigung des Reaktionsproduktes erfolgt vorzugsweise indem der Reaktionsansatz in Ether aufgenommen, mehrfach mit Wasser ausgeschüttelt und die Etherphase anschließend getrocknet und eingeengt wird. Das Reaktionsprodukt wird vorzugsweise durch Kristallisation oder chromatographische Methoden weiter gereinigt.

Der zweite Schritt der Reaktion kann erfolgen, indem das Reaktionsprodukt in einem inerten organischen Lösungsmittel wie z.B. Dioxan, Toluol, Xylole usw. weiter erhitzt wird, bei einer Temperatur, die ebenfalls empirisch zu ermitteln ist. Das erhaltene Produkt, das vorzugsweise durch chromatographische Methoden und/oder Umkristallisation aus organischen Lösungsmitteln z.B. Hexan zu reinigen ist, ist ebenfalls isolierbar und neu. Erfindungsgegenstand ist demzufolge weiterhin eine Verbindung der Formel VIII

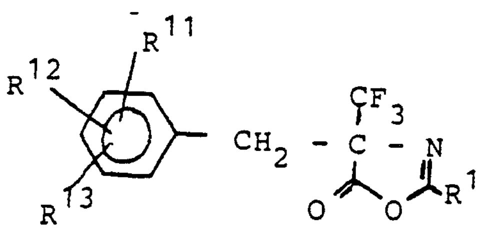

(VIII)

in der $R^1$ die zu Formal VII und $R^{11}$ bis $R^{13}$ die zu Formel VI genannten Bedeutungen haben.

Die erhaltenen 2-Oxazolin-5-one können weiter umgesetzt werden, indem sie mit einem mit Wasser mischbaren Lösungsmittel, vorzugsweise Tetrahydrofuran, Dioxan oder Aceton gelöst und mit verdünnter Säure z.B. 0,5 n HCl versetzt werden. Nach beendeter Hydrolyse kann des Reaktionsprodukt eingeengt werden, vorzugsweise bis zur Trockne und anschließend mit einem Lösungsmittel, bzw. Lösungsmittelgemisch, vorzugsweise Ether/Wassergemisch, aufgenommen werden. Nach mehrmaliger Extraktion der organischen Phase mit Wasser kann die organische Phase getrocknet werden. Nach Entfernung des Lösungsmittels kann das Produkt, falls nötig, weiter gereinigt werden, z.B. durch Kristallisation oder chromatographische Methoden.

Erfindungsgegenstand sind weiterhin die Verwendung der erfindungsgemäßen Verbindungen als Enzyminhibitoren, insbesondere für Aminosäure-Decarboxylasen, Aminosäure-Transaminasen sowie Arzneimittel mit einem Gehalt an erfindungsgemäßen Verbindungen oder erfindungsgemäß hergestellten Verbindungen neben üblichen Hilfs- und/oder Trägerstoffen und gegebenenfalls weiteren Wirkstoffen.

Anhand nachfolgender Beispiele wird das Herstellungsverfahren erläutert:

**Beispiel 1**

(Umsetzung einer Verbindung der Formel II mit Allyl- und Propargylalkoholen)

Zu einer Lösung von 10 mmol 5-Fluor-2-phenyl-4-(trifluormethyl)-oxazol (X = F) in 30 ml wasserfreiem Dioxan gibt man die äquimolare Menge des entsprechenden Allyl- bzw. Propargylalkohols und 1,12 g (20 mmol) festes KOH. Nach kurzer Induktionsperiode erfolgt eine deutliche Erwärmung. Zeigt die $^{19}F$-NMR-Analyse vollständigen Umsatz an, wird der Reaktionsansatz mit 10 ml Wasser versetzt und nach vollständiger Öffnung des Lactonrings ($^{19}F$-NMR-Analyse) zur Trockne eingedampft. Der Rückstand wird in Wasser/Ether (1:1) aufgenommen und die Etherphase 2mal mit Wasser extrahiert. Die vereinigten Wasserphasen werden mit halbkonzentrierter HCl angesäuert (pH 1-2) und mit Ether 3mal extrahiert. Die Etherauszüge werden über Natriumsulfat getrocknet. Nach Entfernung des Lösungsmittels wird der Rückstand aus Ether/Pentan umkristallisiert.

**Die nachfolgenden Verbindungen wurden analog Vorschrift 1 hergestellt.**

| Beispiel | Verbindung | Fp.(°C) |
|---|---|---|
| 1 | 2-(N-Benzoylamino)-2-trifluormethyl-pent-4-ensäure | 141 |
| 2 | 2-(N-Benzoylamino)-2-trifluormethyl-hex-4-ensäure | 144-145 |
| 3 | 2-(N-Benzoylamino)-2-trifluormethyl-hept-4-ensäure | 123-124 |
| 4 | 2-(N-Benzoylamino)-2-trifluormethyl-oct-4-ensäure | 84- 85 |
| 5 | 2-(N-Benzoylamino)-2-trifluormethyl-hepta-4,6-diensäure | 135-137 |
| 6 | 2-(N-Benzoylamino)-3-methyl-2-trifluormethyl-pent-4-ensäure | 167-168 |
| 7 | 2-(N-Benzoylamino)-2-trifluormethyl-3-vinyl-hexansäure | 105-107 |
| 8 | 2-(N-Benzoylamino)-3-phenyl-2-trifluormethyl-pent-4-ensäure | 184 |
| 9 | 2-(N-Benzoylamino)-3,3-dimethyl-2-trifluormethyl-pent-4-ensäure | 164-165 |
| 10 | 2-(N-Benzoylamino)-2-(3-isopropenyl-6-methyliden-cyclohexyl)-3,3,3-trifluorpropansäure | 76- 78 |
| 11 | 2-(N-Benzoylamino)-2-trifluormethyl-penta-3,4-diensäure | 113 (Zers.) |
| 12 | 2-(N-Benzoylamino)-2-trifluormethylhepta-3,4-diensäure | 96- 98 |
| 13 | 2-(N-Benzoylamino)-5-methyl-2-trifluormethyl-hepta-3,4-diensäure | 110-112 |
| 14 | 2-Amino-2-trifluormethyl-pent-4-ensäure | 180 (Zers.) |

**Beispiel 2**

Zu einer Lösung von 10 mmol 5-Fluor-2-phenyl-4-trifluormethyloxazol in 30 ml wasserfreiem Dioxan gibt man die äquimolare Menge eines Allylalkohols, dessen Doppelbindung in ein heteroaromatisches System integriert ist und 1,12 g (20 mmol) festes KOH. Nach kurzer Induktionsperiode erfolgt eine deutliche Erwärmung. Zeigt die $^{19}F$-NMR-Analyse vollständigen Umsatz an, wird die Reaktionsmischung mit 30 ml Wasser versetzt und nach vollständiger Ringöffnung ($^{19}F$-NMR-Analyse) zur Trockne eingedampft. Der Rückstand wird mit Wasser/Ether (1:1) aufgenommen und die Etherphase 2mal mit Wasser extrahiert. Die vereinigten wässrigen Phasen werden mit halbkonzentrierter HCl angesäuert (pH 1- 2) und 3 mal mit Ether extrahiert. Die Etherauszüge werden über Natriumsulfat getrocknet. Nach Entfernung des Lösungsmittels im Vakuum wird der Rückstand aus Chloroform/Hexan umkristallisiert.

**Die nachfolgenden Verbindungen wurden analog Beispiel 2 hergestellt:**

| Beispiel | Verbindung | Fp. (°C) |
|---|---|---|
| 15 | 2-(N-Benzoylamino)-2-(2-methyl-3-furyl)-3,3,3-trifluorpropansäure | 87- 89 |
| 16 | 2-(N-Benzoylamino)-2-(2-methyl-3-thienyl)-3,3,3-trifluorpropansäure | 187-188 |

**Beispiel 3**

Zu einer Lösung von 10 mmol 5-Fluor-2-phenyl-4-trifluormethyl-oxazol in 30 ml wasserfreiem Dioxan gibt man die äquivalente Menge des entsprechenden Benzylalkohols und 1,12 g (20 mmol) festes KOH. Nach kurzer Induktionsperiode erfolgt leichte Erwärmung. Zeigt die $^{19}F$-NMR-Analyse vollständigen Umsatz an, wird der Reaktionsansatz zur Trockne eingedampft und der Rückstand in 30 ml Wasser aufgenommen und 3mal mit Ether extrahiert. Die Etherauszüge werden über Natriumsulfat getrocknet, anschließend wird das Lösungsmittel entfernt. Das 5-Benzoxy-2-phenyl-4-trifluormethyl-oxazol wird umkristallisiert oder bei Bedarf säulenchromatographisch gereinigt.

Das 5-Benzoxy-2-phenyl-4-trifluormethyl-oxazol wird in einem inerten organischen Lösungsmittel aufgenommen und solange bei entsprechender Temperatur erhitzt bis die ($^{19}F$-NMR-Anlayse kein Ausgangsprodukt mehr zeigt. Die Reinigung der erhaltenen 4-Benzyl-2-phenyl-4-trifluormethyl-2-oxazolin-5-one erfolgt durch Säulenchromatographie und anschließende Umkristallisation aus Hexan.

Die so erhaltenen 2-Oxazolin-5-one werden in Dioxan gelöst und mit 0,5 n HCl versetzt. Nach beendeter Hydrolyse ($^{19}F$-NMR-Analyse) wird das Reaktionsgemisch zur Trockne eingedampft und anschließend mit einem Ether/Wasser-Gemisch aufgenommen. Nach 3maliger Extraktion der Etherphase mit Wasser wird diese über Natriumsulfat getrocknet. Nach Entfernung des Lösungsmittels fällt das Produkt analysenrein an.

**Die nachfolgend aufgeführten Verbindungen wurden nach Beispiel 3 hergestellt:**

| Beispiel | Verbindung | Fp. (°C) |
|---|---|---|
| 17 | 5-Benzoxy-2-phenyl-4-trifluormethyl-oxazol | 76 |
| 18 | 4-(4-Methoxybenzoxy)-2-phenyl-4-trifluormethyl-oxazol | 68-69 |
| 19 | 5-(3,4-Methylendioxybenzoxy)-2-phenyl-4-trifluormethyl-oxazol | 68-69 |
| 20 | 5-(3,4,5-Trimethoxybenzoxy)-2-phenyl-4-trifluormethyl-oxazol | 70-71 |
| 21 | 5-(4-Fluorbenzoxy)-2-phenyl-4-trifluormethyl-oxazol | 55-56 |
| Beispiel | Verbindung | Fp. (°C) |
| 22 | 5-(4-Chlorbenzoxy)-2-phenyl-4-trifluormethyl-oxazol | 76-77 |
| 23 | 5-(4-Brombenzoxy)-2-phenyl-4-trifluormethyl-oxazol | 90 |
| 24 | 5-(4-Nitrobenzoxy)-2-phenyl-4-trifluormethyl-oxazol | 115-116 |
| 25 | 5-(1-Phenylethoxy)-2-phenyl-4-trifluormethyl-oxazol | Öl |
| 26 | 5-(4-Pyridylmethoxy)-2-phenyl-4-trifluormethyl-oxazol | 77-78 |
| 27 | 4-Benzyl-2-phenyl-4-trifluormethyl-3-oxazolin-5-on | Öl |
| 28 | 4-(4-Methoxybenzyl)-2-phenyl-4-trifluormethyl-oxazolin-5-on | 56-58 |
| 29 | 4-(3,4-Dimethoxybenzyl)-2-phenyl-4-trifluormethyl-oxazolin-5-on | 128 |
| 30 | 4-(3,4,5-Trimethoxybenzyl)-2-phenyl-4-trifluormethyl-oxazolin-5-on | 96-97 |
| 31 | 4-(4-Brombenzyl)-2-phenyl-4-trifluormethyl-oxazolin-5-on | Öl |
| 32 | 2-(N-Benzoylamino)-2-benzyl-3,3,3-trifluorpropansäure | 145(Zers.) |

## Ansprüche

1. α-Trifluormethylaminosäure-Derivate der Formel I und deren an der Carbonsäurefunktion und/oder an der Aminofunktion geschützte Formen

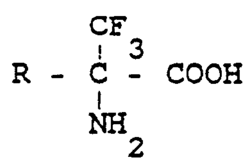

(I)

in der R ein Kohlenwasserstoffrest mit 3 - 20 C-Atomen ist, der auch mit Heteroatomen und/oder verschiedenen funktionellen Gruppen substituiert sein kann, wobei die Verbindungen ausgenommen sind, die einen Rest R tragen, der eine -CHY-Z Gruppe ist und Z eine Phenyl- oder substituierte Phenylgruppe ist und Y niederes Alkyl, Wasserstoff oder eine OH Gruppe ist, sowie die Verbindungen der Formel I in der R eine $-CH_2-CH_2-CH_2-NH_2$ Gruppe ist.

2. Verfahren zur Herstellung von α-Trifluormethylaminsäure-Derivaten der Formel I und deren an der Carbonsäurefunktion und/oder an der Aminofunktion geschützten Formen

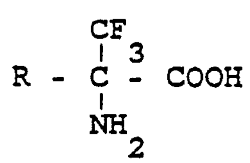

(I)

in der R ein gesättigter oder einfach oder mehrfach ungesättigter Kohlenwasserstoffrest mit mindestens 3 C-Atomen ist, der auch mit Heteroatomen und/oder verschiedenen funktionellen Gruppen substituiert sein kann, dadurch gekennzeichnet, daß man ein in 5-Stellung substituiertes 4-Trifluormethyl-oxazol der Formel II,

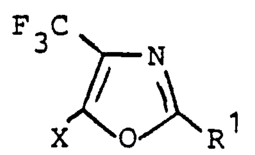

(II)

in der X ein Halogen ist und $R^1$ ausgewählt ist aus der Gruppe Aryl, Heteroaryl, Alkyl, Cycloalkyl bzw. denen mit schwach nukleophilen Heteroatomen und/oder funktionellen Gruppen substituierten Derivaten, mit einem $\beta$-$\gamma$-ungesättigten Alkohol umsetzt und mit der erhaltenen Verbindung eine Umlagerungsreaktion durchführt.

3. Verfahren nach Anspruch 2, dadurch gekennzeichnet, daß der $\beta$-$\gamma$-ungesättigte Alkohol ein Allylalkohol, Propargylalkohol oder ein Benzylalkohol ist.

4. Verfahren nach Anspruch 2 oder 3 zur Herstellung von Verbindungen der Formel III

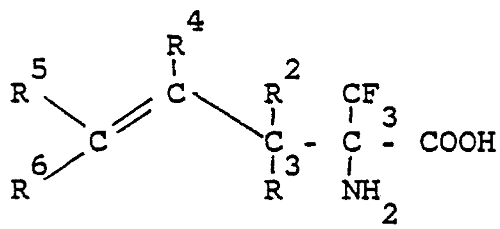

(III)

in der $R^2$ - $R^6$ Wasserstoff, geradkettige oder verzweigte Alkyl-, Alkenyl-, Alkinyl-, Acyl-, Aryl-, Aralkyl-, Heteroaryl- oder heterocyclische Gruppen mit bis zu 10 C-Atomen sein können, wobei die Substituenten Halogenatome tragen können und/oder durch funktionelle Gruppen wie OH-, -SH und/oder -$NH_2$ in geschützter oder ungeschützter Form substituiert sein können, und wobei die einzelnen Reste $R^4$ und $R^5$, $R^2$ und $R^3$, $R^5$ und $R^6$, $R^2$ und $R^5$, $R^2$ und $R^6$, $R^3$ und $R^5$ sowie $R^3$ und $R^6$ durch Verbrückung ein Ringsystem bilden können, das carbocyclischer, heterocyclischer und/oder aromatischer Natur sein kann, wobei jede einzelne Verbrückung bis zu 10 C-Atome aufweisen kann und zwei oder mehrere Ringsysteme annelliert oder miteinander verknüpft sein können und wobei die Verbrückungen substituiert sein können mit Alkyl- oder Alkenylgruppen mit bis zu 5 C-Atomen.

5. Verfahren nach Anspruch 4, dadurch gekennzeichnet, daß die Substituenten $R^2$ bis $R^6$ in Formel III Wasserstoff, geradkettige oder verzweigte Alkyl- oder Alkenylgruppen mit 1 - 5 C-Atomen oder eine Arylgruppen sind.

6. Verfahren nach den Ansprüchen 4 oder 5, dadurch gekennzeichnet, daß die Substituenten $R^2$ bis $R^6$ Wasserstoff, geradkettige oder verzweigte Alkyl- oder Alkenylgruppen mit 1 - 3 C-Atomen sind.

7. Verfahren nach den Ansprüchen 2 - 4, dadurch gekennzeichnet, daß mindestens eine Verbrückung zwischen den Substituenten $R^2$ bis $R^6$ besteht, wobei die Verbrückung bis zu 6 C-Atome, eine oder mehrere Doppelbindungen aufweisen kann, eines oder mehrere der Brücken C-Atome durch N, O oder S und die Verbrückung bzw. Verbrückungen durch einen oder mehrere Alkyl-, Alkenyl- oder Arylsubstituenten mit bis zu 5 C-Atomen substituiert sein können.

8. Verfahren nach den Ansprüchen 2, 3 oder 7, dadurch gekennzeichnet, daß die Substituenten $R^3$ und $R^6$ eine Verbrückung der Formel IV bilden

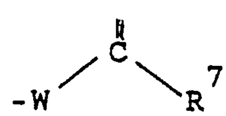

(IV)

wobei $R^7$ Wasserstoff oder eine Alkylgruppe mit bis zu 4 C-Atomen darstellt und W Kohlenstoff, Stickstoff, Sauerstoff oder Schwefel ist.

9. Verfahren nach Anspruch 2 zur Herstellung von Verbindungen der Formel V

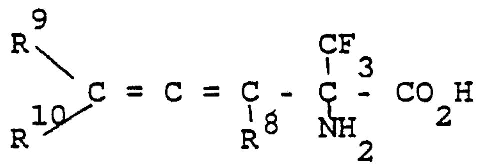

(V)

in der die Substituenten $R^8$ bis $R^{10}$ Wasserstoff, geradkettige oder verzweigte Alkylreste mit bis zu 10 C-Atomen, heterocyclische Reste oder Aryl- oder Hetroarylreste sein können, die mit Halogenatomen und/oder mit den Schutzgruppen versehenen Funktionen -OH, -SH, $-NH_2$, $-CO_2H$, $P(OH)_3$ und/oder $-SO_3H$, substituiert sein können, wobei die Reste $R^9$ und $R^{10}$ auch zusammen eine Verbrückung bilden können.

10. Verfahren nach den Ansprüchen 2 oder 3 zur Herstellung von Verbindungen der Formel VI

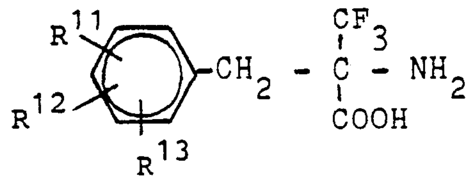

(VI)

in der $R^{11}$, $R^{12}$ und $R^{13}$ unabhängig voneinander Wasserstoff, Halogen (Chlor, Brom, Fluor), Nitro-, Alkoxy- oder Alkylgruppen mit bis zu 5 C-Atomen oder Phenylgruppen sein können, wobei zwei dieser Substituenten auch zusammen ein Ringsystem bilden können.

11. Verbindungen der Formel VII

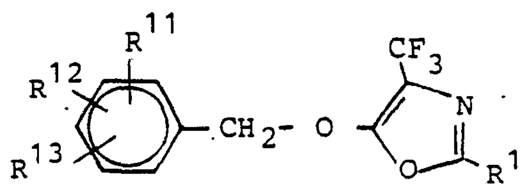

(VII)

in der $R^1$ die in Anspruch 2 und $R^{11}$ bis $R^{13}$ die in Anspruch 10 genannten Bedeutungen haben.

12. Verbindungen der Formel VIII

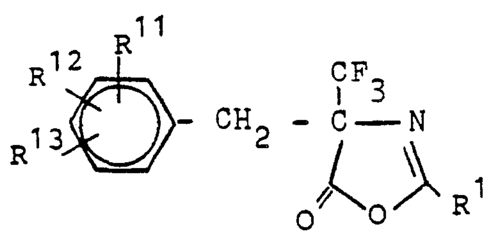

(VIII)

in der $R^1$ die in Anspruch 2 genannten Bedeutungen hat und die substituenten $R^{11}$ bis $R^{13}$ die in Anspruch 10 genannten Bedeutungen haben.

13. Verwendung von Verbindungen nach Anspruch 1, 11 oder 12 oder von Verbindungen, die nach einem Verfahren gemäß einem oder mehreren der Ansprüche 2 - 10 hergestellt wurden als Enzyminhibitoren.

14. Arzneimittel, gekennzeichnet durch einen Gehalt an Verbindungen nach Anspruch 1, 11 oder 12 oder an Verbindungen, die nach einem Verfahren gemäß einem oder mehreren der Ansprüche 2 - 10 hergestellt wurden, neben üblichen Hilfs- und/oder Trägerstoffen und gegebenenfalls weiteren Wirkstoffen.

Europäisches Patentamt

# EUROPÄISCHER RECHERCHENBERICHT

Nummer der Anmeldung

EP 89105659.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | US - A - 3 046 300 (MEYER SLETZINGER et al.) * Spalte 1, Zeilen 41-56 * -- | 13,14 | C 07 C 103/84<br>C 07 C 101/28<br>C 07 C 102/00<br>C 07 C 99/00<br>C 07 D 263/42<br>C 07 D 307/54<br>C 07 D 333/24<br>A 61 K 31/195<br>A 61 K 31/42 |
| P,X | CHEMICAL ABSTRACTS, Band 110, Nr. 21, 22. Mai 1989, Columbus, Ohio, USA K. BURGER et al. "Synthesis of alpha-trifluoromethyl substituted aspartic acid and its derivatives" Seite 807, Zusammenfassung-Nr. 193 359v & J. Fluorine Chem. 1988, 41(3), 429-33 -- | 1-6 | |
| P,X | CHEMICAL ABSTRACTS, Band 109, Nr. 15, 10. Oktober 1988, Columbus, Ohio, USA K. BURGER et al. "A simple route to 2-substituted 3,3,3-trifluoroalanine derivatives" Seite 757, Zusammenfassung-Nr. 129 631e & Angew. Chemie 1988, 100(6), 860-1 -- | 1-6,9 | **RECHERCHIERTE SACHGEBIETE (Int. Cl.4)**<br>C 07 C 103/00<br>C 07 C 101/00<br>C 07 C 102/00<br>C 07 C 99/00<br>C 07 D 263/00<br>C 07 D 333/00<br>C 07 D 307/00 |
| P,X | CHEMICAL ABSTRACTS, Band 109, Nr. 7, 15. August 1988, Columbus, Ohio, USA V.A. SOLOSHONOK et al. "Fluorine-containing amino acids. III. Alpha-Trifluoromethyl amino acids." Seite 176, Zusammenfassung-Nr. 55 185p & Zh. Org. Khim. 1987, 23(11) 2308-13 | 1 | |
| P,Y | -- | 13,14 | |

Der vorliegende Recherchenbericht wurde für alle Patentansprüche erstellt.

| Recherchenort | Abschlußdatum der Recherche | Prüfer |
|---|---|---|
| WIEN | 03-07-1989 | KÖRBER |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

Europäisches Patentamt

# EUROPÄISCHER RECHERCHENBERICHT

Nummer der Anmeldung

-2-
EP 89105659.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 106, Nr. 19, 11. Mai 1987, Columbus, Ohio, USA<br>S.N. OSIPOV "Synthesis and C-alkylating properties of 2-(benzenesulfohylimino)-3,3, 3-trifluoropropionic acid methyl ester"<br>Seite 665, Zuammenfassung-Nr. 156 213q<br>& Izv.Akad.Nauk SSSR, Ser. Khim.1986, (6), 1384-7<br>-- | 1 | |
| X | CHEMICAL ABSTRACTS, Band 106, Nr. 23, 8. Juni 1987, Columbus, Ohio, USA<br>V.A. SOLOSHOHOK et al. "Trifluoropyruvic acid (methoxycarbonyl)imine"<br>Seite 690, Zusammenfassung-Nr. 195 861u<br>& Zh.Org.Khim.1986,22(6), 1335-7<br>-- | 1 | RECHERCHIERTE SACHGEBIETE (Int. Cl.4) |
| X | CHEMICAL ABSTRACTS, Band 108, Nr. 21, 23. Mai 1988, Columbus, Ohio, USA<br>S.N. OPISOV et al. "Reactions of methyl 2-imino-3,3,3-tri-fluoropropionates with CH- and PH-acids"<br>Seite 718, Zusammenfassung-Nr. 186 849k<br>& Izv.Akad. Nauk SSSR, Ser. Khim.1987,(5), 1185-8<br>-- | 1 | |
| A | CHEMICAL ABSTRACTS, Band 92, Nr. 3, 21. Jänner 1980, Columbus, Ohio, USA<br>J.W. KELLER et al. "3,3,3- | 13 | |

Der vorliegende Recherchenbericht wurde für alle Patentansprüche erstellt.

| Recherchenort | Abschlußdatum der Recherche | Prüfer |
|---|---|---|
| WIEN | 03-07-1989 | KÖRBER |

KATEGORIE DER GENANNTEN DOKUMENTEN
X : *von besonderer Bedeutung allein betrachtet*
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | Trifluoro-2-aminoisobutyrate: a mechanism-based inhibitor of Pseudomonas cepacia alpha--dialkylamino acid transaminase" Seite 285, Zusammenfassung-Nr. 17 854d & Biochem. Biophya. Res. Commun. 1979, 90(4), 1104-10 -- | | |
| A | CHEMICAL ABSTRACTS, Band 106, Nr. 19, 11. Mai 1987, Columbus, Ohio, USA M. EL-SAID MUSTAFA "Trifluoropyruvic and hydrate in heterocyclic synthesis. Part III. A novel synthesis of 4-(trifluoromethyl)oxazolones and related compounds" Seite 677, Zusammenfassung-Nr. 156 322z & Heterocycles 1986, 24(6), 1541-7 ---- | 11,12 | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| WIEN | 03-07-1989 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument